# EUROPEAN PATENT SPECIFICATION

(11) **EP 2 688 626 B1**
(45) Date of publication and mention of the grant of the patent: **15.08.2018**
(21) Application number: 12761312.3
(22) Date of filing: 23.03.2012
(51) Int. Cl.: A61M 16/00, B01D 53/02, B01D 53/56, A61M 16/10, B01D 53/04

(54) **SYSTEM FOR COLLECTING NITROUS OXIDE IN EXHALATION AIR**
SYSTEM ZUM SAMMELN VON STICKOXID IN AUSGEATMETER LUFT
SYSTÈME POUR COLLECTER DE L'OXYDE NITREUX DANS DE L'AIR D'EXPIRATION

(30) Priority: 24.03.2011 SE 1130018; 30.03.2011 SE 1130019; 30.03.2011 US 201161469369 P; 30.03.2011 US 201161469381 P; 07.04.2011 SE 1130026
(43) Date of publication of application: 29.01.2014
(73) Proprietor: Medclair AB, 784 33 Borlänge (SE)
(72) Inventor: SZABÓ, István, S-795 96 Boda Kyrkby (SE)
(74) Representative: Ehrner & Delmar Patentbyrå AB
(86) International application number: PCT/SE2012/000043
(87) International publication number: WO 2012/128694

(56) References cited:
- EP-A2- 0 284 227
- WO-A1-2010/071538
- WO-A2-2009/095601
- WO-A2-2009/095605
- WO-A2-2009/095611
- US-A- 4 004 585
- US-A- 5 231 980
- US-A1- 2005 072 298
- US-A1- 2005 072 423
- US-A1- 2006 174 878
- US-A1- 2009 095 296
- US-A1- 2010 052 293
- US-B1- 6 716 271
- US-B2- 7 178 522

## Description

### TECHNICAL FIELD

The present invention relates to a system for collecting nitrous oxide in air exhaled (= exhalation air) by one or more individuals and subsequently delivering the nitrous oxide to an apparatus for further processing. The invention comprises the system as such, a method of using the system, an adsorption unit containing a reversible nitrous oxide adsorbent, and a pool of such units.

### BACKGROUND TECHNOLOGY

Nitrous oxide is an air pollutant which is considered at least 300 times more effective than carbon dioxide as a "green house gas". The gas is considered hazardous for people exposed to it during work (e.g. doctors, dentists, nurses etc). Occupational health limits have been set to 25 ppm. Cost-effective and convenient apparatuses, systems and methods for reducing discharge of the gas to the atmosphere are likely to be imperative in the future.

Within health care units, nitrous oxide is used within surgery, dental care, maternity care during delivery etc due to its anaesthetic and analgesic effect. The patient is allowed to inhale a gas mixture (= inhalation air) in which the main components are nitrous oxide, typically in concentrations ≥ 10%, such as ≥ 20% and/or ≤ 80%, such as ≤ 70 % (v/v) and oxygen. The levels of nitrous oxide and oxygen are essentially the same for inhalation air and exhalation air. The levels of moisture (H₂O) and carbon dioxide are increased in exhalation air compared to inhalation air. When an enhanced anaesthetic effect is desired, the mixture also contains a gaseous anaesthetic agent, as a rule in concentrations ≤ 10%, such as ≤ 5% or ≤ 2 % with typically levels being in the range of 0.25-3 %, such as 0.5-2 % (v/v). Suitable anaesthetic agents have been selected amongst volatile halo-containing organic compounds, e.g. halo-containing hydrocarbons, halo-containing ethers etc, and other volatile and/or gaseous organic compounds which are capable of exerting an anaesthetic effect, for instance anaesthetic hydrocarbons not containing halo substituents.

At larger health care units exhalation air containing nitrous oxide is typically handled in a waste gas handling system which is common for several rooms/patients. In these kind of systems the exhaled air is typically diluted with ambient air (e.g. 10-50 times) and finally treated for removal of nitrous oxide at the health care unit and/or passed into ambient atmosphere.

Catalytic removal of nitrous oxide from exhaled air from patients has been discussed:
DE 42087521 (Carl Heyer GmbH), DE 4308940 (Carl Heyer GmbH), EP 2165756 (Linde AG), US 4,259,303 (Kuraray Co., Ltd), WO 2011075033 (Nordic Gas cleaning AB), WO 20101071538 (Nordic Gas Cleaning AB), WO 2010081642 (Linde AG), WO 2010081643 (Linde AG), WO 2006059506 (US 7,608,232; Showa Denko KK), WO 2002026355 (US 7,235,222, US 7597858; Showa Denko KK), JP publ No. 55-031463 (Kuraray Co Ltd), JP publ No. 56-011067 (Kuraray Co Ltd), JP publ No. 2006230795 (Asahi Kasei Chemicals Corp).

An experimental study on adsorption of nitrous oxide in ventilation air by the use of zeolites is given in *"*Removal of laughing gas from air by the use of zeolites" IVL commission for Stockholms Läns Landsting, www.sll.se: Report 2007-03-08.

The publications given above are mainly focusing on removal of nitrous oxide by catalytic decomposition, adsorption or compression/condensation.

At smaller health care units and/or during minor treatments requiring short-time inhalation of nitrous oxide it is neither cost effective nor convenient to use the equipments used for larger units. For smaller units and minor treatments it becomes inconvenient and expensive with a common system for handling and/or decomposition of waste anaesthetic gases. The levels of nitrous oxide to deal with will be inherently higher which is associated with its own problems. Typical smaller health care units and minor treatments are e.g. ambulances, dentists, private doctors, local health centres, acute clinics etc. Previously suggested apparatuses and methods have typically been adapted for use close to a patient and are in many cases based on adsorption; see for instance WO 2009095601, WO 2009095605 and WO 2009095611 (all of Air Liquid). See also EP 0284227 (Union Carbide), EP 1356840 (Siemens-Elema), and EP 1797942 (Univ Delft), international patent application PCT/SE2011/000202 (Nordic Gas Cleaning AB),US 3941573 (Chapel) and US 5928411 (Drägerwerk). Mobile docking arrangements as generically defined in this specification are also included in the mobile adsorbtion units described in WO 2009095601, WO 2009095605 and WO 2009095611.

See also *Removal of laughing gas from air at dentists by the use of zeolites"* Stockholms läns landsting SSL - Occupational Health, www.sll.se: Report 2010-06-10: "
EP 2165756 (Linde AG) expressly discusses close-to patient use and temperature regulation during exothermic decomposition of nitrous oxide.

There is a need for more cost-effective and convenient systems and methods for collecting nitrous oxide from exhaled air of single patients at health care units and subsequent further processing of nitrous oxide to environmentally acceptable end products. These systems should a) be adapted for use close to a patient, b) be flexible and simple to manufacture and use, c) support favourable total energy balance, d) support discharge of acceptable levels of harmful substances to the environment (e.g. nitrous oxide and NOₓ (x = 1 och/eller 2)) etc.

### OBJECTS

The main object of the invention comprises to provide convenient and cost effective systems and methods for handling of nitrous oxide collected from exhaled air as discussed above.

### DRAWINGS

- **Figure 1**: illustrates an adsorption unit to be used in the system. The unit has two separate inlet ports and two separate outlet ports, i.e. in total four ports.
- **Figure 2**: illustrates an adsorption unit which has two ports each of which is a combined inlet/outlet port and docking arrangements comprising scales as sensors for measuring amount of nitrous oxide caught in the unit. In **figure 2a** the adsorption unit is connected to a face mask for inhaling nitrous oxide (adsorption mode). In **figure 2b** the adsorption unit is connected to an apparatus for further processing of nitrous oxide (desorption mode).
- **Figure 3**: illustrates a mobile adsorption unit having two ports for inlet/outlet of gas and temperature sensors for measuring amount of nitrous oxide caught in the unit.
- **Figure 4**: illustrates the preferred variant of adsorption units developed during the priority year. The double headed arrow designates the flow direction of desorbing gas during desorption mode (desorption flow, air). The single headed arrow designates the flow direction of air exhaled by a patient during adsorption mode (adsorption flow).
- **Figure 5**: illustrates a preferred catalytic decomposition apparatus developed during the priority year and intended to be used in the inventive system.

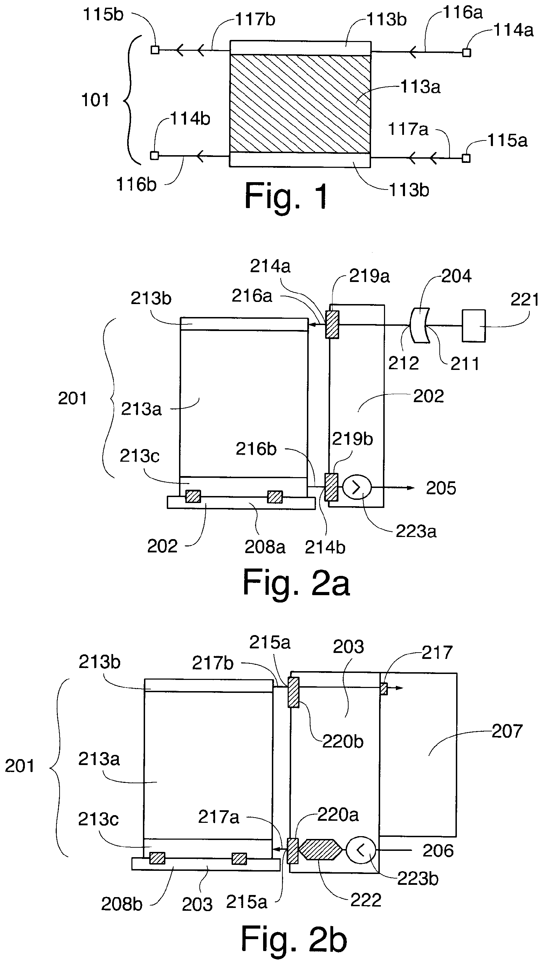

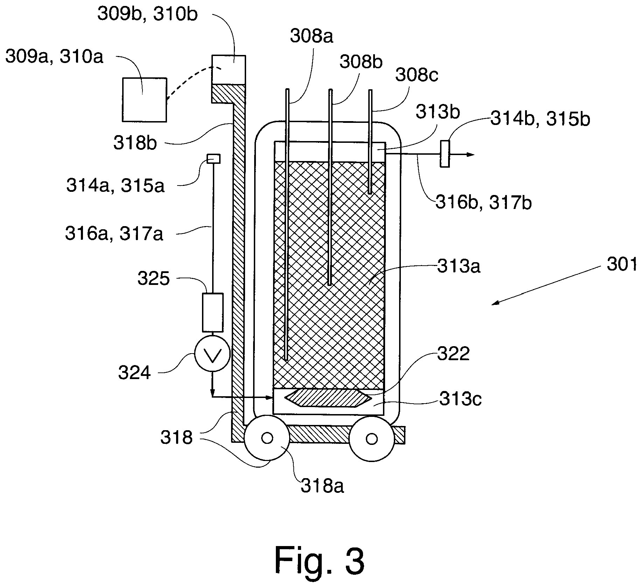

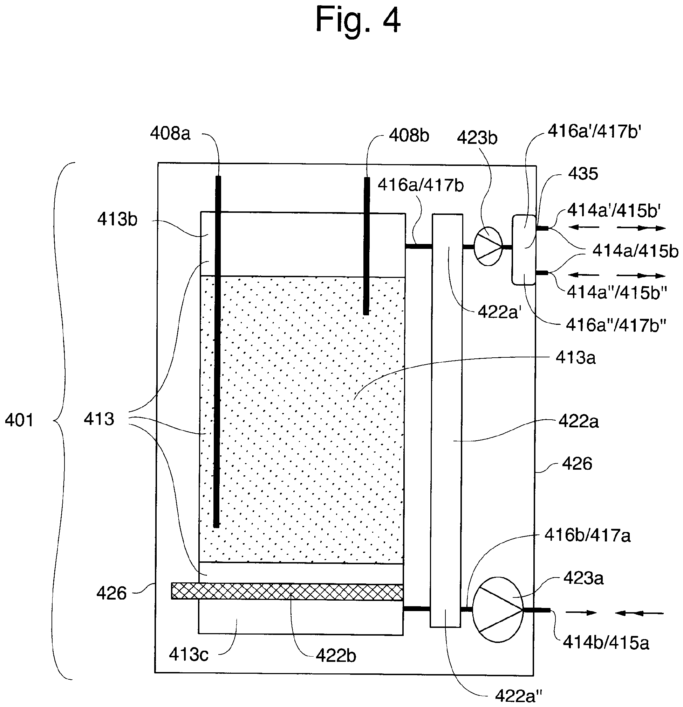

Flow directions are indicated with arrows (in fig 1 with > for adsorption and >> for desorption). Reference numerals in the figures comprise three digits. The first digit refers to the number of the figure and the second and third digits to the specific item. Corresponding items in figures 1-4 have as a rule the same second and third digits. Figure 3 represents modes considered to be the most advantageous at the priority filing date and figures 4-5 represent modes that were preferred at the international filig date. See also preferred variants discussed below.

### INVENTION

The inventor has realized that the objects given above can be met for the systems and methods that are generally defined under *Technical Field* by including a pool of one, two or more through-flow nitrous oxide adsorption units (101,201,301) which are mutually replaceable (if there are two or more of them in the pool), together with
A) a docking arrangement DA_{ads} (202) for connecting an adsorption unit (201) of the pool to
   a) the face mask (204) used by an individual exhaling nitrous oxide, and
   b) a waste recipient (205) for exhaled air processed in the unit (i.e. depleted with respect to nitrous oxide), and
B) a docking arrangement DA_{des} (203) for connecting an adsorption unit (201) of the pool to
   a) a source for a desorbing gas (206), and
   b) an apparatus (207) for further processing of nitrous oxide, and
C) optionally a measuring arrangement comprising one or more sensors (208a,b,308a,b,c) for measuring the amount of nitrous oxide caught in an adsorption unit (201,301), and
D) optionally a logging arrangement (309a+b) with a memory (310a+b) for storing data reflecting working efficiency for at least the latest time of use for the individual adsorption units (301) of the pool.

The individual adsorption units are mobile. At least docking arrangement DA_{ads} for adsorption may be mobile and have simple design. See below.

The expression "for connecting" means both "is connected" and "is capable of being connected". The connection may be indirect or direct where indirect includes via a functionality, e.g. via valves, heaters, flow changing functions, filters etc and direct means via simple connections and/or extension conduits without any particular functionality other than transportation of gas/fluid. This applies all throughout the specification if not otherwise indicated.

The expression "mutually replaceable" means that corresponding parts of individual adsorption units of the pool have the same geometrical fit for the system, e.g. with respect to geometry of inlet ports and outlet ports, connections to docking arrangements, connections to sensors etc. It also means that the individual adsorption units are mobile between a docking arrangement DA_{ads} and a docking arrangement DA_{des}, i.e. between different possible locations of individuals inhaling nitrous oxide and/or between such a location and an apparatus for further processing of nitrous oxide. Corresponding definition also applies to variants of the inventive system comprising several DA_{abs} and/or several DA_{des}.

The expression "further processing" encompasses e.g. decomposition, pooling, storing and the like of nitrous oxide adsorbed in an adsorption unit. Pooling and/or storing are possibly combined with condensation/compression/ fractionation. There are a number known apparatus and methods for performing further processing; see the publications discussed above and recently filed SE 1130018-3 filed March 24, 2011 and US 61/469,381 filed March 30 2011 and corresponding international patent application filed in parallel with this application (all three with the title *Apparatus for treating gas* with Nordic Gas Cleaning AB as proprietor and Istvan Szábó as inventor).

### SYSTEM ASPECT OF THE INVENTION (1^{st} aspect)

The first aspect of the invention is a system for carrying out a method comprising the steps of:
I) collecting nitrous oxide in air exhaled by an individual inhaling/exhaling nitrous oxide via a facemask arrangement (204), and
II) delivering the nitrous oxide collected in step (I) to an apparatus (207) for further processing of nitrous oxide collected in step (I).

Inhalation/exhalation of nitrous oxide is done via a face mask arrangement (204) (further on called "face mask") which comprises an inlet ILₘₐₛₖ (211) for inhalation and an outlet OLₘₐₛₖ (212) for exhalation plus the face mask as such together with various arrangements supporting and facilitating undisturbed breathing when the mask is used. The velocity for exhaled air leaving a face mask arrangement is typically within the interval of 15-40 L/min, such as 20-30 L/min.

The main characteristic feature is that the system comprises a pool containing one, two or more adsorption units (201,201',202" etc) together with arrangements A+B, preferably combined with C and/or D.

### THE POOL OF ADSORPTION UNITS

In addition to nitrous oxide adsorption units the pool may also contain other adsorption units, e.g. for adsorbing anaesthetic agents, moisture etc. The term "adsorption unit" will further on only refer to "mobile and mutually replaceable and reversible nitrous oxide adsorption units", if not otherwise indicated by the context.

An adsorption unit (101,201,301) has a through-flow adsorption chamber (113,213,313) which contains a through-flow nitrous oxide reversible adsorbent (113a,213a,313a) placed in the chamber typically leaving a gap (113b,c,213b,c,313b,c) devoid of adsorbent at each end of the chamber. The unit also has:
i) An inlet port IP_{ads} (114a,214a,314a) for inlet of an adsorption flow containing exhalation air, i.e. nitrous oxide. This port is connectable to an outlet port OLₘₐₛₖ (212) of a face mask arrangement (204). The connection may be direct or indirect.
ii) An outlet port OP_{ads} (114b,214b,314b) for outlet of the adsorption flow. The flow exiting this port is depleted in nitrous oxide. It is discharged to a waste recipient (205). The waste recipient may be ambient atmosphere. This port is in preferred variants directly connectable to ambient atmosphere (redundant connection). In other variants the connection to ambient atmosphere is indirect via functionalities as illustrated below.
iii) An inlet port IP_{des} (115a,215a,315a) for inlet of a desorption flow which contains a desorbing gas (purge gas). The port is connectable to a source for desorbing gas (206). This source is preferably ambient atmosphere or may alternatively be in the form of a storage container containing e.g. pressurized air, pressurized nitrogen gas etc. The source may include an arrangement for pre-processing the desorbing gas, e.g. a heater, a flow changing function, a drier etc. In other words this port may be directly or indirectly connectable to the source as such.
iv) An outlet port OP_{des} (115b,215b,315b) for outlet of the desorption flow. This flow releases (= desorbs) nitrous oxide from the adsorbent and thus will contain nitrous oxide when exiting this port. The port is directly or indirectly connectable to the inlet port IPₐₚₚ (217) of an apparatus (207) for further processing of nitrous oxide desorbed from adsorption units of the pool.

The inlet port IP_{ads} (114a,214a,314a) and the outlet port OP_{ads} (114b,214b,314b) of a unit define the flow direction Flow_{ads} of the adsorption flow through the chamber/unit/adsorbent. The two ports are placed at opposite ends/parts of the chamber/unit/adsorbent and accordingly define an upstream end/part and a downstream end/part in relation to the adsorption flow.

The inlet port IP_{des} (115a,215a,315a) and the outlet port OP_{des} (115b,215b,315b) of the unit define the flow direction Flow_{des} for the desorption flow through the chamber/unit/adsorbent. The two ports are placed at opposite ends/parts of the chamber/unit/adsorbent and accordingly define an upstream end/part and a downstream end/part in relation to the desorption flow.

The flow directions Flow_{ads} and Flow_{des} may have the same or opposite directions through the chamber (413). The inlet ports IP_{ads} and IP_{des} (114a,214a,314a and 115a,215a,315a) for the adsorption flow and the desorption flow, respectively, may be i) at opposite ends or ii) at the same end of the chamber/unit/adsorbent, where (i) means opposite directions for the flows and (ii) the same direction for the flows. The same applies for the outlet ports OP_{ads} and OP_{des} (114b,214b, 314b and 115b,215b,315b). A convenient arrangement is that ports pair-wise coincide, e.g.
a) *opposite flow directions*: inlet port IP_{ads} (114a,214a,314a) coincides with outlet port OP_{des} (115b,215b,315b), and/or outlet port OP_{ads} (114b,214b,314b) coincides with inlet port IP_{des} (115a,215a,315a) with preference for "and", and
b) *same flow direction:* inlet port IP_{ads} (114a,214a,314a) coincides with inlet port IP_{des} (115a,215a,315a), and/or outlet port OP_{ads} (114b,214b, 314b) coincides with outlet port OP_{des} (115b,215b,315b) with preference for "and".

The directions in space of the two flows can be vertical or horizontal. Vertical directions are preferred and include a) vertically upwards with an angle between the flow direction and the vertical line being within ± 45° preferably 0°, and b) vertically downwards with a corresponding interval of 180° ± 45°, preferably 180°. Intervals of the same widths are valid for downwardly and upwardly directed horizontal flows.

For adsorption/desorption under flow conditions in general it is considered to be optimal to reverse the flow direction when switching from adsorption to desorption. This principle also applies to adsorption/desorption in the present invention. However, the system of the invention might be simpler, more convenient, more cost-effective etc to use with the same flow direction for adsorption and desorption, i.e. the adsorption unit is constructed as illustrated in figure 3 (coinciding inlet ports (314a,315a), coinciding outlet ports (314b,315b) coinciding inlet conduits (316a,317a), coinciding outlet conduits (316b,317b) etc). See below under the heading *Desorption part of the flow regulating arrangement.*

The gaps/empty spaces (113b,c,213b,c,313b,c) cover the ends of the adsorbent in order to support even distribution of adsorption flow/exhalation air and/or desorbing flow/gas through the adsorbent (113,a,213a,313a). Placed at an upstream end the gap is an example of a distributor function. Placed at a downstream end the gap is an example of a collector function. As illustrated in figure 3, the gap/empty space (313c) next to an inlet port IP_{des} (315a) for the desorption flow may in preferred variants contain a heating function (322).

The adsorption unit may also comprise an inlet conduit (116a,216a,316a,117a,217a,317a) for each inlet port (114a,214a,314a,115a,215a,315a) and/or an outlet conduit (116b,216b,316b, 117b,217b,317b) for each outlet port (114b,214b,314b, 115b,215b,315b) for gas flow communication between the chamber and the inlet ports and the outlet ports, respectively. These conduits may coincide forming a common conduit in the same manner as the inlet/outlet ports with the same preferences as for the ports. A common outlet/outlet conduit, inlet/outlet conduit, or inlet/inlet conduit may comprise that the common conduit divide into two branch conduits each of which ends in an inlet or outlet port according to the two functions of the common conduit. This kind of branching is typically associated with a valve function permitting separate opening of each of the two branch conduits while at the same time leaving the common conduit open. Theoretically the branching may be the other way round, i.e. with both branches ending at the decomposition chamber (313).

The adsorption unit (201,201,301) may also contain other functionalities as discussed below, e.g. carrier functions (318a,318), an air inlet (325), a flow changing function (324), one or more valves, one or more sensors (308a,b,c), parts of a logging arrangement (309b,310a) such as a memory etc.

The total volume of the adsorbent (113a,213a,313a) should be sufficient for two or more separate medical treatments with administration of nitrous oxide lasting for about 15 minutes each (mean administration times). A typical range is 10-50 separate treatments. This means that the amount of adsorbent per adsorption unit should be sufficient for a total effective collecting time in the interval of > 30 min with a typical interval of 50 - 750 min. For adsorbents of essentially the same bulk density (660-740 g/L), particle size (1.5-2.5 mm) and specific capacity (0.075g N₂O/g adsorbent) as the adsorbent used in the experimental part, this means that suitable volumes may be found in the interval of 5 - 30L per adsorption unit with a weight in the interval of roughly from 1-2 kg to 20 kg. For adsorbents having other densities, particle sizes and capacities per unit volume, suitable intervals for weights and volumes may be found by adapting these general guidelines to the actual densities, particle sizes and specific capacities of these other adsorbents.

The outer dimensions of the chamber including isolation, walls and the like are typically: a) the height (along the flow direction) is typically > 10 cm such as within the interval of 20 - 180 cm, and b) the cross-sectional area (orthogonal to the flow direction) corresponds to a circular cross-sectional area with a diameter > 5 cm, such as within the interval of 10 - 100 cm, such as 10 - 80 cm. The shape of the cross-sectional area of the unit as well as of the chamber and the adsorbent is preferably circular.

### Adsorbent

The adsorbent (113a,213a,313a) is typically in the form of a porous bed. This bed preferably comprises a bed of packed particles, preferably porous particles, e.g. comprising chemistry and/or micropores in a size range classifying the material as a molecular sieve. Suitable micopore sizes are found in the interval of 1-12 Angstrom for removing nitrous oxide from a gas stream which contains exhalation air, i.e. nitrous oxide together with oxygen and typically also moisture (H₂O) and/or carbon dioxide. The particles shall have sizes such that the void volume between the particles when packed to a bed defines a through-passing macroporous system which permits flow transport of inhalation air and desorbing gas through the bed. Suitable particle sizes for particulate materials are found within the interval of 0.5 - 10 mm, with preference for within 1 - 5 mm (diameters). This includes that a minor part of the particle material may be particles with sizes outside these ranges, e.g. < 25% or < 10 % or < 5% or < 1 %. The particles are preferably spheroidal, i.e. rounded including in particular beaded forms such as in the form spheres.

The adsorbent (113a,213a,313a) may alternatively be a macroporous monolith or plug exhibiting micropores of the sizes given above for particles.

Suitable adsorbent materials are found amongst materials of the above-mentioned type having a capacity for adsorbing nitrous oxide in the interval of 0.025 - 0.25 g of N₂O/g adsorbent material.

Suitable adsorbent material should be stable under the temperatures applied during adsorption and desorption, i.e. from around 15-20°C to the upper temperatures given for desorption.

The adsorbent (113a,213a,313a) is reversible with respect to adsorption/desorption. In other words it can be regenerated after adsorption to give an adsorbent having sufficient adsorbing capacity for nitrous oxide and through-flow capacity for being reused in the system of the invention. The regeneration is carried out by passing a desorbing gas, preferably heated, through the adsorbent to desorb nitrous oxide. The adsorbent material should preferably allow for regeneration at least 5 or at least 10 or at least 15 or at least 20 times with a retained capacity of ≥ 50 %, such as ≥ 60 % or ≥ 75% of the initial capacity for removing, binding or adsorbing nitrous oxide from exhalation air.

An important class of adsorbent material are zeolites which may be either natural zeolites or more preferably modified zeolites, e.g. with native Na⁺ ions being replaced with Ca²⁺ ions. Suitable materials can be obtained from among others Merck, Darmstadt, Germany (Moleculare sieve 0.5 nm) and Grace Davison, Grace GmbH, Worms, Germany (Molecular sieves MS S 624).

Further guidelines for selecting adsorbent materials to be used in the invention are found in literature related to adsorption of nitrous oxide from industrial off-gases (e.g. US 6,080,266 UOP LLC, US 6,719,827 Air Products and Chemicals Inc, US 20100071552 Virani et al etc) and from exhalation air (e.g. WO 2009095601, WO 2009095605 and WO 2009095611 all of Air Liquid, US 3,941,573 Chapel, US 5,928,411 Drägerwerk, US 3,941,573 Chapel). See also Stockholms läns landsting www.sll.se Report 2007-03-08 *"Removal of laughing gas from air by the use zeolites",* and Report 2010-06-10: *"Removal of laughing gas from air at dentists by using zeolites"* (both reports compiled by IVL))

Reversible nitrous oxide adsorbents (113a,213a,313a) for which there is a measurable parameter which changes as a function of the proceeding of the adsorption of nitrous oxide are likely to have a great potential for use in the invention. By selecting this kind of adsorbent together with an appropriate sensor (308a,b,c) it may be possible to measure when the proceeding of the adsorption front has reached one or more predetermined position along the flow direction in the adsorbent during an ongoing adsorption (position = longitudinal position). Every such position will represent utilized capacity (= a particular degree of utilization of the capacity) or of remaining capacity to utilize (= available capacity) when the front reaches the position. The outlet end of the adsorbent will mean that 100% has been utilized with no remaining capacity to utilize. Other predetermined positions may stand for e.g. ≥ 50 %, ≥ 75 %, ≥ 85 %, ≥ 90 % of the initial capacity is utilized (or ≤ 50 %, ≤ 25 % ≤ 15 %, ≤ 10 % of the initial capacity remains to be utilized or is still available). A finding that the adsorption front has reached a certain position can be used for alerting personnel handling the system to disconnect the unit and initiate subsequent desorption and further processing of desorbed nitrous oxide. Changes in the time needed for the adsorption zone to reach a certain predetermined position between repetitive occasions of use of the same adsorbent/unit will be indicative about the latest status of the working efficiency of the adsorbent/unit. It can be envisaged that this might be used for determining when an adsorbent/adsorption unit needs be discarded/repacked with fresh adsorbent material.

The adsorption unit may also have a memory (310b) which is part of the memory (310a+b) of a logging arrangement (309a+b) of the system of the invention as discussed under *D. Logging arrangement* below. Other parts of the an adsorption unit of the invention are
Every adsorption unit of the pool typically comprises a carrier function (318) comprising e.g. a) wheels (318a) and/or one or more handles (318b) fixedly mounted on the vessel containing the chamber/adsorbent, or b) a cart for carrying the vessel, which contains the adsorbent, and other parts of the unit as discussed above and below.

### A. DOCKING ARRANGEMENT FOR ADSORPTION (DA_{ads})

Docking arrangement DA_{ads} (202) is associated with the adsorption of nitrous oxide. The arrangement is adapted for connecting a face mask (204) of an individual exhaling nitrous oxide to a waste recipient (205) for exhaled air depleted in nitrous oxide via an adsorption unit (201). A typical docking arrangement DA_{ads} (202) comprises:
a) an upstream 1^{st} connection C1_{ads} (219a) for connecting the inlet port IP_{ads} (214a) of an adsorption unit (201) to the outlet OLₘₐₛₖ (212) of a face mask (204), and
b) optionally a downstream 2^{nd} connection C2_{ads} (219b) for connecting the outlet port OP_{ads} (214b) of the same adsorption unit (201) to the waste recipient (205) for exhaled air depleted in nitrous oxide, preferably to ambient atmosphere, or alternatively to a waste storage vessel for exhaled air processed in the adsorbent unit.

Exhaled air depleted in nitrous oxide thus may be discharged directly from the outlet port OP_{ads} (214b) to ambient atmosphere. This can render the connection C2_{ads} (219b) obsolete, i.e. preferred variants are devoid of this optional connection.

The system can have one, two or more of docking arrangement DA_{ads} (202) for use at different locations of a health care unit. The arrangement DA_{ads} is preferably mobile in the sense that it can be disconnected/reconnected from/to a face mask and transported with or with the face mask connected to between patients and/or locations where there is a need for administering nitrous oxide. See also *Developments during the priority year* at the end of this specification where we describe a simple variant which can be transported together with the adsorprtion unit. Compare also WO 2009095601, WO 2009095605 and WO 2009095611 discussed above.

### B. DOCKING ARRANGEMENT FOR DESORPTION (DA_{des})

Docking arrangement DA_{des} (203) is associated with desorption of nitrous oxide by passing a desorbing gas through an adsorption unit. The arrangement (203) is thus adapted for connecting a source (206) of desorbing gas to an apparatus for further processing of nitrous oxide via an adsorption unit (201) (which have been charged with nitrous oxide in docking arrangement DA_{ads} (202)). A typical docking arrangement DA_{des} comprises
a) optionally an upstream 1^{st} connection C1_{des} (220a) for connecting the inlet port IP_{des} (215a) of an adsorption unit (201) to a source (206) for desorbing gas, and
b) a downstream 2^{nd} connection C2_{des} (220b) for connecting the outlet port OP_{des} (215b) of the same adsorption unit (201) to the inlet port IPₐₚₚ (217) of an apparatus (207) for further processing of nitrous oxide released by the desorbing gas when it passes through the adsorption unit.

The source for desorbing gas has been discussed above together with the inlet port IP_{des}.(215a). Air from ambient atmosphere (206) may be used directly as desorbing gas without any preprocessing. The need for connection C1_{des} (220a) may then be obsolete, i.e. preferred variants are devoid of this optional connection.

Either one or both of the docking arrangements DA_{ads} (202) and DA_{des} (203) may comprise a flow changing function (223a,b), a heating function (222), valve functions, a sensor (208a,b) etc.

See also *Developments during the priority year* at the end of this specification.

### C. MEASURING ARRANGEMENT

The system is preferably associated with a measuring arrangement which comprises one or more sensors (208a,b,308a,b,c). These arrangement/sensors are capable of measuring amounts of nitrous oxide retained in the individual adsorbents during and/or after adsorption. The sensors may be based on measuring changes in weight (208a,b) of an adsorbent/unit, changes in available and/or utilized capacity (308a,b,c) and/or other parameters changing as a consequence of the adsorption, e.g. the position of the adsorption front (308a,b,c) during ongoing adsorption, changes in level of nitrous oxide in the adsorption flow downstream of the adsorbent (e.g. break-through or the level downstream of the adsorbemt relative the level upstream of the adsorbent), changes in temperature (308a,b,c) in the adsorbent due to evolution of heat during adsorption (see above) etc. Thus a typical sensor used in the measuring arrangement may be a weight sensor (208a,b), a spectrometric sensor, a temperature sensor (308a,b,c) etc. A spectrometric sensor is illustrated with an IR sensor in the Experimental Part.

In principle every parameter for which a measurable change also indicates a measurable change in amount of nitrous oxide retained on the adsorbent can be used. Sensors measuring consumption of nitrous oxide, such as number of exhalations/inhalation (sensor: a pulse meter), lowering of amount of nitrous oxide in the source (221) of nitrous oxide etc, could also be included in the measuring arrangement. With respect to number of inhalations/exhalations, individuals most likely will have to be grouped, e.g. according to weight, sex, age, health status, etc where every group have a relatively narrow interval for volume of inhaled air per inhalation/exhalation.

In one variant of the measuring arrangement, there is a sensor (208a,b) which is common for several adsorption units (201) . Sensors (208a,b) in arrangements of this kind may be based on weighing (scales, changes in weight) and may be part of A) the apparatus for further processing (207), B) docking arrangement DA_{ads} (202), and/or C) docking arrangements DA_{des} (203). Other possible sensors (common to several adsorption units (201)) are: Sensors based on consumption of nitrous oxide (measured e.g. at the source (221) of nitrous oxide) and number of inhalations/exhalations (measured at the face mask (204, for instance), spectrometric sensor, e.g. an IR sensor placed in the gas flow downstream of C2_{ads} (219b) (provided this optional connection is present) etc.

In another variant, every individual adsorption unit (308a,b,c) is associated with its own sensor. This kind of arrangement is preferably based on sensors measuring temperature changes or other changes in the adsorbent due to adsorption, and/or changes in the level of nitrous oxide in the adsorption flow downstream of the adsorbent but still within the adsorption unit (primarily only break-through). Other possible sensor types to be used on individual adsorption units can be found among those mentioned above for the measuring arrangement in general, for instance weight sensors, such as load cells, spectrometric sensors, such as IR sensors, etc

A potentially interesting variant comprises one, two or more temperature sensors (308a,b,c) placed at different downstream positions in an adsorbent (313a) for which adsorption of nitrous oxide is exothermic; see above under *The pool of adsorption units,* subheading *Adsorbents.* This implies a variant of the present invention which comprises that
A) The adsorbents (313a) in the adsorption units (301) comprise adsorbent material for which the adsorption of nitrous oxide is exothermic and leads to measurable temperature increases where adsorption is ongoing. A heated front which is moving downstream within the adsorbent during adsorption will be indicative of the ongoing adsorption and of the position of the adsorption front at different times.
B) One or more temperature sensitive sensors (308a,b,c) which
   a) are capable of measuring changes in temperature in the adsorbent caused by adsorption of nitrous oxide, and
   b) are placed at different predetermined longitudinal positions between the inlet end and the outlet end of the adsorbent (313a).

One predetermined position can be in the central part and second position close to the outlet of the adsorbent (313a). This corresponds to about 50 % and about 10 % of the total capacity still being available downstream of the first and second position, respectively. Future results are likely to show that either upward flow or downward flow is to be preferred.

### D. LOGGING ARRANGEMENT

The system of the invention typically comprises a logging arrangement (309a+b) for
a) keeping track of changes in functional status of the individual adsorption units (301) and
b) alerting when to discard and/or replace a particular unit with a freshly prepared unit, i.e. when the unit has become too bad for further regeneration.

This implies that the system of the invention also may include an appropriate alarm function for this purpose.

A central part of the logging arrangement is a memory (310a+b) for storing
a) a unique identification code for every adsorption unit, i.e. for the individual units, and
b) data reflecting working efficiency for at least the latest time of use of the individual units, optionally including also corresponding data for a freshly prepared adsorbent/unit, and preferably
c) predetermined (= preset) limit values for disqualifying/qualifying an adsorption unit (the individual adsorption units) for repetitive use (i.e. values which preferably correspond to data of (b)).

Stored data of type (b) refer to values obtained by the measuring arrangement and include also data which are derived from such values. In particular adsorption capacity data and/or flow property data (e.g. pressure drop at the working conditions) may be stored.

Data of type b) include capacity data relating to adsorption of nitrous oxide and/or flow capacity data and/or sum of running times for adsorption for at least the latest time of use for the individual adsorbents of the pool etc, and possibly also corresponding initial values for freshly prepared adsorbents of the pool.

The memory of the arrangement may comprise local memories (310b) (one or more per adsorption unit (301)) and/or a central memory (310a) separate from the local memories. A local memory may be physically attached to or physically separated from its adsorption unit and/or typically contains the unique identification code for the unit possibly together with other information collected at least for the latest time of use of the unit, or a reference to such information in a central memory for instance via the identification code. The central memory (310a) typically includes the necessary information for keeping track of important changes in unity-specific data of the kind mentioned in the previous paragraphs. Alternatively the central memory only contains the identification code and collects all or a part of the unit-specific data from the appropriate local memories by referring to this code.

The local memories and/or the central memory may be in the form of a conventional log-book or label with the stored information in typed-out form, or as readable and/or writeable electronic memories or the like. Transmission of information between the memories may be wire-less or via wires.

### HEATING ARRANGEMENT

The heating arrangement comprises one or more functions (222,322) each of which is capable of heating the adsorbent in every adsorption unit to effectuate quick and efficient desorption when a unit is connected for desorption. Heating may take place via direct heating of the adsorbent, e.g. micro-wave heating, or by preheating the desorbing gas upstream of the adsorbent.

In one main variant there is a heating function (322) in the individual adsorption units (301). This heating function is then preferably placed between the inlet port IP_{des} (315a) for the desorption flow and the adsorbent (313a), preferably in the upstream end of the chamber (313), e.g. in the gap (313c), or in the inlet conduit (317a) for the desorption flow.

In another main variant the heating function (222) is common to several adsorption units. The position for the heating function (222) is then preferably upstream of the connection C1_{des} (220a) of docking arrangement DA_{des} (203).

In the case there is a flow changing function FCF_{des} downstream of a heating function, there should be a cooling function between the heating function and the flow changing function. In order for the heated desorbing gas to effectuate desorption, this means that both the cooling function and the flow changing function FCF_{des} should be placed downstream of the adsorbent (213). This in particular applies if the flow changing function FCF_{des} is a blower since conventional blowers are normally not designed to resist preheated gas flows. At the priority filing it was felt that this cooling function should be avoided on the adsorption units (201) and it was therefore at that time preferred to place such a cooling function and the flow changing function on docking arrangement DA_{des} (203) or in the apparatus for further processing (207). This view has been changed during the priority year. See *Developments during the priority year* at the end of this specification (Figures 4-5).

A heating function (222,322) shall be capable of heating the through-passing desorbing gas and/or the adsorbent (313a) to a temperature enabling efficient release of nitrous oxide from the adsorbent. Suitable temperatures depend on the desorbing gas, adsorbent material etc, and are typically found in the interval of ≤ 400°C, such as 100-400°C or 100-250°C. The effect of the heating function at least for preheating should be within the interval of 150-2500 W with preference for within 200-500 W. The heater may be gradually adjustable with respect to effect and is preferably in the form of an electrical heating element possibly supported by a heat exchanger as described under *Developments during the priority year* at the end of this specification.

### FLOW REGULATING ARRANGEMENTS

The system of the invention is associated with a flow regulating arrangement which comprises two parts: An adsorption part and a desorption part. The arrangement comprises various flow functions, such as flow changing functions FCF, valves, vents to air etc. The major part of these functions are part of the apparatus (207) for further processing, the face mask arrangement (204), the source (206) for desorbing gas and/or the waste recipient (205) all of which as such are well known in the field. The remaining flow functions, if any, are present on the adsorption units (201) and/or the docking arrangements DA_{ads} (202) and/or DA_{des} (203). A general rule is to place as few flow functions as possible on the mobile adsorption units.

Flow changing functions FCF are used for initiating, stopping, increasing and/or decreasing flow velocity. In the adsorption part they are called FCF_{ads} and in the desorption part FCF_{des}. Flow changing functions are preferably blowers and/or preferably frequency controlled and/or preferably gradually adjustable with respect to flow velocity. This applies to in principle every flow changing function which is part of the flow regulating arrangement and is independent of position in the arrangement.

### Adsorption part of the flow regulating arrangement

A predicted preferred variant of the invention presumes that the flow of exhalation air (=adsorption flow) leaving a face mask arrangement (204) is sufficient for the flow to pass through the adsorption unit (201). However, this will require a sufficiently stable adsorption flow (exhaled air) both during the treatment of an individual and between treatments and/or individuals. The system of the invention is therefore likely to become more versatile if a flow changing function FCF_{ads} is included in the adsorption part of the flow regulating arrangement, e.g. as indicated in the variants illustrated by figures 2 and 3. In other words the function FCF_{ads} can be present in the face mask arrangement (204), docking arrangements DA_{ads} (202), the mobile adsorption units (201), and/or the arrangement for the waste recipient (205). This has been elaborated further during the priority year. Se below.

In one variant a flow changing function FCF_{ads} (223a) is common to several adsorption units (201). This means that the function is present a) on the face mask arrangement (204), b) on the docking arrangements DA_{ads} (202) upstream of the connection C1_{ads} (219a) and/or downstream of the connection C2_{ads} (219b) and/or c) on the arrangement for the waste recipient (205). Preferred positions for this variant are (b) or (c).

In other variants of the invention, individual adsorption units comprise a flow changing function FCF_{ads} (324). This means that the flow changing function FCF_{ads} (324,423) when present on the unit (301,401) is always placed between the inlet port IP_{ads} (314a,414a) and the outlet port OP_{ads} (315b,415,b). Typical positions are in the inlet or outlet conduits (116a,216a,316a and 117b,217b,317b, respectively).

A variant thought to be advantageous at the priority date comprises a flow changing function FCF_{ads} (324) placed upstream or downstream of the adsorbent (313a) in combination with an air inlet (325) placed upstream of the flow function FCF_{ads} (324). The preferred positions for the air inlet (325) and the function FCF_{ads} (324) are in the inlet conduit (316a) as illustrated in figure 3. The air inlet (325) may be designed as a) an air inlet conduit or b) a circular gap defined by two coaxial tubes of different inner diameters, for instance. The two coaxial tubes in (b) are part of the inlet conduit (316a) and placed end-to end with the thinner tube possibly being partly inserted into the thicker tube. The circular gap is defined as the space between the inner wall of the thicker tube and the outer wall of the thinner tube. This arrangement may be used for maintaining the flow velocity given by a flow changing function FCF_{ads} (324) at a desired value through the adsorbent without risk for disturbing the function of a connected face mask. The arrangement may be combined with a flow sensor (flow meter or pressure sensor) placed in the inlet conduit (316a) downstream of the air inlet (325) but upstream of the flow function FCF_{ads} (324).

The arrangement described in the preceding paragraph may also be used for the desorption flow, see figure 3 where the inlet port IP_{ads} (314a) coincides with the inlet port IP_{des} (315a) and the inlet conduit (316a) coincides the inlet conduit (317a). The flow changing function FCF (324) is thus common for the adsorption flow and the desorption flow. It may then be appropriate that the air inlet (325) is associated with a valve function permitting closing of the air inlet during desorption when the common inlet port (314a,315a) is connected to a source for desorbing gas, e.g. via a docking arrangement DA_{des} (203). Alternatively the air inlet may be designed to have a dual function and work as a) an inlet port IP_{des} for desorbing gas such as air from ambient atmosphere during desorption or b) an air inlet during adsorption as described in the preceding paragraph. The arrangement with an air inlet in this latter variant thus may comprise a common inlet/inlet conduit which in the upstream direction divides into two branches each of which is ending in an inlet port (inlet port IP_{ads} and IP_{des}, respectively). One of these inlet ports work as an inlet port IP_{des} during desorption and an air inlet during adsorption, i.e. has the dual function described previously, while the other one is an inlet port IP_{ads} for air containing nitrous oxide during adsorption and is preferably closed during desorption. The branching is typically associated with a valve function comprising
a) a first position for the adsorption mode permitting flow through the branch which contains both inlet port IP_{ads} and the air inlet to the chamber while the branch which contains the inlet port IP_{des} is closed,
b) a second position for the desorption mode permitting flow through the branch which contains the inlet port IP_{des} to the chamber while the branch which contains the other port (= IP_{ads}) is closed.

At the end of the priority year a simpler alternative to the inlet/outlet function illustrated in figure 3 (preceding two paragraphs) had been developed. See *Developments during the priority year* at the end of this specification (figures 4-5).

### Desorption part of the flow regulating arrangement

In one variant a flow changing function FCF_{des} (223b) for desorption flow may be common for several adsorption units. This variant means that the flow changing function may be placed
a) upstream of the connection C1_{des} (220a) of the docking arrangement DA_{des} (203) for desorption, i.e. on the docking arrangement or as part of the source for desorbing gas, and/or
b) downstream of the connection C2_{des} (220b) of the same docking arrangement as in (a), i.e. on the docking arrangement or as part of the apparatus for further processing (207).

This is illustrated in figure 2b with the flow changing function FCF_{des} (223b) placed according to a) on docking arrangement DA_{des} (203).

In other variants of the invention, individual adsorption units comprise a flow changing function FCF_{des} which may be present between an inlet port IP_{des} (115a,215a,315a) and the adsorbent (113a,213a,313a) and/or between the same adsorbent and an outlet port OP_{des} (115b,215b,315b). The preference is for in the inlet or outlet conduits (116a,216a,316a and 117b,217b,317b, respectively).

In figure 3 there is illustrated an advantageous variant with a flow changing function FCF_{des} (324) in an inlet conduit (316a) for desorption flow which is coinciding with an inlet conduit (317a) for adsorption flow (FCF_{des} = FCF_{ads} (324)). This is further discussed above under *Adsorption part of the flow regulating arrangement.*

See also *Developments during the priority year* at the end of this specification (Figures 4-5).

### Other functionalities in the system.

The system may also contain a pool of one or more mutually replaceable adsorption units for removing an anaesthetic agent present together with nitrous oxide in an anaesthetic gas (not shown). The system then encompasses the appropriate docking arrangement on which these units may be replaceable inserted/displaced. This docking arrangement may be placed upstream of the connection C1_{ads} (219a) of the docking arrangement DA_{ads} (202), such as within the docking arrangement. Alternatively the chamber or unit with an adsorbent for an anaesthetic agent may be present on the adsorption unit carrying a nitrous oxide adsorbent. This chamber then may be combined with the chamber for the nitrous oxide adsorbent or as a separate chamber/adsorbentThe adsorbent for the anaesthetic agent is then preferably placed upstream of the nitrous oxide adsorbent.

The system may also comprise a function for removing particles (filter function, not shown) and/or a function for removing moisture (moisture adsorbent, e.g. silica material, not shown) from the incoming adsorption flow (exhalation air). When these functions are present in the system they may be connected (= placed) upstream of the adsorption unit for nitrous oxide and the possible adsorption unit for removal of an anaesthetic agent. A filter function and/or a moisture adsorbent are preferably placed upstream of the connection C1_{ads} (219a) of docking arrangement DA_{ads} (202) and/or upstream of the connection C1_{des} (220a) of docking arrangement DA_{des} (203), and preferably as a part of a docking arrangement. In the case adsorption units for an anaesthetic agent are included in the inventive system, a particle filter function and a moisture adsorbent, if present, should be placed upstream such extra adsorption units.

### METHOD ASPECT OF THE INVENTION (2^{nd} aspect)

This aspect is a method in which the system described above is used for the purpose discussed for the system aspect of the invention. The method comprises the steps of:
i) providing the system as described above,
ii) connecting a nitrous oxide adsorption unit (201) of the pool via docking arrangement DA_{ads} (202) to an individual exhaling nitrous oxide,
iii) allowing exhalation air to pass through the adsorption unit (201) until the saturation of the adsorbent (313a) has reached a predetermined value based on
   a) a starting total capacity value for a freshly prepared adsorbent placed in the adsorption unit, or
   b) possible reductions in this value (i.e. the capacity value of a)) based on the latest prior use of the adsorption unit,
iv) transferring the adsorption unit from docking arrangement DA_{ads} (202) to docking arrangement DA_{des} (203) thereby connecting the unit via the connection C1_{des} (220a) on the docking arrangement DA_{des} (203) to a source for desorbing gas (206) and to the apparatus (207) for further processing via the connection C2_{des} (220b) on the same docking arrangement (203),
v) allowing desorbing gas, preferably heated, to pass through the adsorbent (313a) to release nitrous oxide and delivering desorbing gas plus nitrous oxide to the apparatus (207) for further processing until the adsorbed nitrous oxide has been completely desorbed),
vi) optionally maintaining the flow of desorbing gas to pass through the adsorbent (313a), now at ambient temperature, (heating function off) and possibly with the adsorption unit (201) disconnected from further processing of nitrous oxide and preferably with an moisture adsorbent connected upstream of the adsorption unit,
vii) releasing the adsorption unit (201) from docking arrangement DA_{des}, and
   a) reconnecting this adsorption unit (201), or
   b) connecting another adsorption unit (201') of the pool
   to docking arrangement DA_{ads} (202) while returning the previously used adsorption unit (201) to the pool, i.e. presuming that alternative (b) is selected,
viii) optionally repeating the sequence of steps (ii)-(vii.a) one or more times with the same adsorption unit, possibly interrupted with the sequence of steps (ii)-(vii.b), until the capacity and/or flow properties for an adsorption unit have deteriorated to a level disqualifying it for further repetitions whereupon the unit is discarded and replaced with a freshly prepared adsorption unit

Preferences in the method are mentioned in the description of the system aspect of the invention.

A preferred method aspect comprises that a unit is disqualified based on capacity and/or flow property data derived from measurements made by the measuring arrangement of the system and/or by general guidelines, e.g. given by the manufacturer. Measurement of capacity and flow properties has been discussed above. General guidelines of interest may have been set up empirically, and typically comprise a) an upper limit for number of regeneration cycles for an adsorption unit/adsorbent, b) a maximum total time for adsorption (sum for all cycles run with a particular adsorption unit), c) the time needed for reaching a predetermined saturation level, d) a minimum available total capacity in absolute amount or in relation to available total capacity before or found after the first time the adsorption unit is used in the inventive system etc.

Measured values as well as predetermined limit values etc are typically stored in the memory of the logging arrangement of the system. See above under *Logging Arrangement*

### THE THIRD ASPECT OF THE INVENTION (ADSORPTION UNITS)

A third aspect of the invention is an adsorption unit as generally defined in original claim 1 with the characteristic features as given in subclaims and elsewhere in this specification.

### DEVELOPMENTS DURING THE PRIORITY YEAR.

We have concluded from developments during the priority year that for the most convenient variants of docking arrangements:
- Docking arrangements DA_{ads} should be mobile. Preferred variants comprise that the outlet port OLₘₐₛₖ of the face mask arrangement corresponds/coincides to/with the connection C1_{ads} of the docking arrangement DA_{ads}. There is no optional connection C2_{ads} since processed gas depleted in nitrous oxide is discharged directly to ambient atmosphere via the outlet port OP_{ads} of the adsorption unit. The face mask as such, possibly together with the tube connecting it to the adsorption unit can preferably be disconnected/reconnected as described above for docking arrangement DA_{ads}, and may or may not be transported between different locations and/or patients.
- Docking arrangement DA_{des} should be part of the apparatus for further processing of nitrous oxide. The connection C2_{des} will be a part of this apparatus and coincide with the inlet port IPₐₚₚ of the apparatus. There is no optional connection C1_{des} since ambient air is directly used as desorbing gas without pre-processing in this preferred variant.

These kinds of docking arrangements are in particular adapted to the variants of adsorption units illustrated by figure 4.

**Figure 4** illustrates the most convenient adsorption unit of the inventive system at the priority date. The figure illustrates both adsorption mode with the adsorption flow (exhaled air) represented as a single headed arrow and desorption flow (desorbing gas) represnted as a double headed arrow. As illustrated in the drawing the two flows have opposite direction with preference for vertical directions and further preference for downward for the adsorption flow and upward for the desorption flow (when the flows are passing through an adsorbent (413a)). The adsorption unit may comprise
i) an inlet/outlet port IP_{ads}/OP_{des} (414a/415b) which is common for the inlet of exhaled air (adsorption flow) containing nitrous oxide and the outlet of desorbing gas (desorption flow) containing desorbed nitrous oxide,
ii) an outlet/inlet port OP_{ads}/IP_{des} (414b/415a) which is common for the outlet of exhaled air depleted in nitrous oxide and the inlet of desorbing gas, preferably ambient airs.

There is an imperative adsorption chamber (413) containing the adsorbent (413a) for nitrous oxide between the ports. This chamber preferably has a gap (413b and 413c) at each end of the bed (413a).

Between the common inlet/outlet port IP_{ads}/OP_{des} (414a/415b) and the chamber (413) there is an inlet/outlet conduit (416a/417b) which is common for exhaled air and desorbing gas in the same manner as the common inlet/outlet port IP_{ads}/OP_{des}. This inlet/outlet conduit may be designed with two common inlet branches (416a'/417b' and 416a"/417a") with corresponding branch inlet ports (414a'/415b' and 414a"/415b", respectively) in order to be adapted to a patient which is alternately inhaling/exhaling a nitrous oxide mixture and oxygen or air via two separate face masks. This kind of branching/merging (435) may be placed on the adsorption unit (401) as illustrated in this figure. Alternatively it is placed upstream of the adsorption unit (not shown), e.g. upstream of the connection C1_{ads} of a docking arrangement DA_{ads}, and is then only used for merging the flow of nitrous oxide with the flow not containing nitrous oxide (oxygen or air). Alternatively the branching/merging may be an integrated part of a face mask (only merging). Upstream refers to the adsorption flow. In other variants of the invention, the common inlet/outlet port IP_{ads}/OP_{des} (414a/415b) may be a single port as illustrated in figure 2 (common inlet/outlet port IP_{ads}/OP_{des} (214a/215b)) or as in figure 3 (common inlet/inlet port IP_{ads}/IP_{des} (314a/315a).

The branching/merging function (435) may be associated with a valve function enabling separate opening of each of the two branches/ports or simultaneous opening of them depending on the number of different flows (one or two) entering the unit and how they are going to be treated in the unit. This valve function, if present, is preferably simple, e.g. separate closing/opening of a desired one of the two inlet/outlet ports (414a'/415b' and 414a"/415b") by a plug or a cover, or a true valve encompassing closing/opening at the merging point or within the branch conduits.

The common outlet/inlet port OP_{ads}/IP_{des} (414b/415a) is typically a single port with a single outlet/inlet conduit (416b/417a) between this port and the adsorption chamber (413). The outlet/inlet conduit (416a/417b) is common for the exhaled air and desorbing gas in the same manner as the common inlet port OP_{ads}/IP_{des}.

There is also a heating arrangement (422a+422b) for heating the desorbing gas before it enters the adsorbent (413a). This arrangement is only used during desorption mode, i.e. at least the heater (422b) is turned on during desorption. This kind of heating arrangement is generally in the invention and comprises:
a) a heat exchanger (422a) in which heat in desorbing gas leaving the adsorption chamber (413a) through its downstream end (gap (413b)) is transferred to desorbing gas which is about to enter the adsorbent (413a) through its downstream end (gap, 413c), and
b) a heater (422b) placed in the upstream end (gap (413c)) of adsorption chamber (413) or in an inlet conduit (416b/417a).

The heat exchanger (422a) will thus exert a cooling function (422a') on desorbing gas downstream of the adsorbent and a heating function (422a") on desorbing gas upstream of the adsorbent. This heat exchanger corresponds to the cooling function disclosed in the priority application. It preferably exerts its heating function (422a") upstream of the heater (422b).

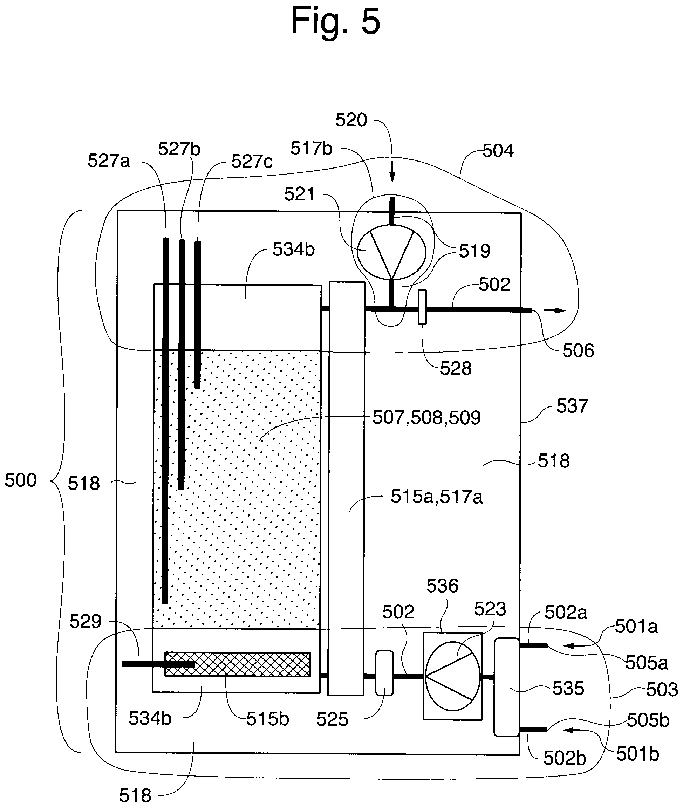

The heat exchanger (422a) is preferably used as a heating complement to the heater (422b) and will
a) optimize the energy balance of the system, and
b) protect flow changing functions FCF, such as a blower, which are placed downstream of the heating arrangement (422a+b) and the adsorbent (413a) from heat induced damages during desorption (downstream refers to desorption flow), for instance flow changing function FCF_{des} (423) and FCF_{des} (523, figure 5).

The flow changing function for controlling desorption flow is advantageously placed on the apparatus for further processing (e.g. FCF_{des} (523) in figure 5, see below) and preferably combined with a flow changing function FCF_{des} (423b) on the adsorption unit (401). An adsorption unit of the invention thus may or may not comprise a flow changing function FCF_{des} (423b). If present on the adsorption unit, the flow changing function (423b) is capable of being turned on during desorption and turned off during adsorption. In the inventive system the controlling FCF_{des} function (523) is thus found downstream of the heating arrangement (422a+b) and thus protected by the cooling function (422a') of the heat exchanger (422a) from heat induced damages during desorption when an apparatus of figure 5 is used for decomposing nitrous oxide desorbed from an adsorption unit of figure 4. The preferred position for FCF_{des} (423b) is for the same reasons downstream of a cooling function (422a') which in turn is placed downstream of the adsorbent (413a).

A flow changing function FCF_{ads} (423a) which is used to secure subpressure and hinder leakage of nitrous oxide at positions upstream of the adsorption unit is preferably placed on the unit (401). The preferred position in the adsorption unit (401) is upstream of the heater (422b) with further preference for also upstream of other parts of the heating arrangement (422a+b) that may be present, e.g. the heating function (422a") of the heat exchanger (422a). This means that the most convenient position is in the common outlet/inlet conduit (416b/417a) through which exhaled air passes before entering the adsorption chamber (413). The flow changing function FCF_{ads} (423a) is capable of being turned off during desorption and turned on during adsorption. It is preferably battery-driven.

The flow changing functions in the two preceding paragraphs are preferably blowers of the same type as discussed as discussed elsewhere in this specification.

The preferred adsorption chamber (413) contains a porous adsorbent (413a) surrounded by ends devoid of adsorbent materials (gaps, 413b and c). One, two or more sensors (408a,b) as part of a measuring arrangement may be placed on the unit, e.g. in the adsorbent (413a), for indicating in real time the saturation degree of the adsorbent during ongoing adsorption of nitrous oxide within the bed. See discussion above. These sensors are preferably temperature sensors which are placed in the adsorbent. There may alternatively be other kinds of sensors for indicating saturation degree or amount of nitrous oxide on the adsorbent as discussed elsewhere in this specification.

The various functional parts of the adsorption unit (401) discussed above are preferably enclosed in a common housing (426). The unit may be equipped with wheels to support mobility. One or two face masks for inhalation of gas containing nitrous oxide and/or oxygen/air, respectively, as well as gas tubes containing these gases may be connected and/or carried to/by a mobile unit which contains the inventive adsorption unit.

During adsorption the common inlet/outlet port IP_{ads}/OP_{des} (414a/415b) is connected to a face mask arrangement used by a patient inhaling nitrous oxide. The flow changing function FCF_{ads} (423a) is turned to secure subpressure upstream of the function and prevent leakage to the environment. Exhaled air is allowed to pass through the adsorbent while nitrous oxide is captured in the adsorbent. Exhaled air depleted in nitrous oxide is discharged to ambient atmosphere through the outlet/inlet port OP_{ads}/IP_{des} (414b/415a). The sensor for saturation (408a) indicates when a predetermined saturation degree of the adsorbent is reached (see discussion above) after which the adsorption unit (401) is disconnected from the face mask arrangement and regenerated by desorption. For temperature sensors as illustrated in figure 4, this means that a warm zone (about 35-60°C) will appear where the adsorption is ongoing and may be used to indicate in real time the degree of saturation of the adsorbent. This zone will move from temperature sensor (408a) towards temperature sensor (408b) when adsorption is ongoing and disappear/reappear depending on if the unit connected to a patient or not. The adsorbent is considered saturated when this zone appears at the downstream end of the adsorbent (e.g. about 5-10 °C above ambient temperature at sensor (408b)).

During desorption the inlet/outlet port IP_{ads}/OP_{des} (414a/415b) of an adsorption unit according to figure 4 is connected to the inlet port (505,505a,b) of an apparatus for further processing (500 in figure 5). Temperature sensors as indicated in figure 4 are used. After the apparatus (500) and the adsorption unit (401) have been started and adapted to predetermined desorption/decomposition conditions, desorbing gas in the form of ambient air is
a) sucked into the adsorption unit (401) via the common outlet/inlet port OP_{ads}/IP_{des} (414b/415a),
b) heated by the heating arrangement (422a+422b) prior to passing through the adsorbent (413c),
c) exiting the adsorbent (413a) together with desorbed nitrous oxide,
d) cooled in the heat exchanger (422b), and
e) finally exiting the unit (401) through its common inlet/outlet port IP_{ads}/OP_{des} (414a/415b) while entering the apparatus for further processing (500 of figure 5) where nitrous oxide is catalytically decomposed to N₂ and O₂.

During desorption the heater (422b) and the flow changing function FCF_{des} (423) on the adsorption unit (401) and the flow changing functions FCF_{des} (523), and FCF (521) and the heating arrangement (515) on the apparatus (500) are turned on. The flow changing functions FCF_{des} (423) and FCF (523), i.e. the desorption flow, is controlled by the flow sensor (525) on the decomposition apparatus to be within presets limits (cable between the adsorption unit and the decomposition apparatus). The sucking force will be the pressure differential caused by the flow changing functions FCF_{des} (523). The flow changing function FCF_{ads} (423) on the adsorption unit (401) is turned off during desorption of nitrous oxide. During desorption the adsorbent will be warmed up by the heater (422b) starting from the end next to the heater (422b) (100-200°C). When the temperature at the sensor (408b) at the opposite end of the adsorbent has reached a predetermined value (about 100 °C) the heater (422b) is turned off. Either one or both of the flow changing functions FCF_{des} (423) and FCF (523) is on for 1-2 additional hours in order to complete desorption (about 40 °C at temperature sensor (408a) at the outlet end of the adsorbent). The heater (422b) is controlled from the apparatus (500) (cable).

**Figure** 5 illustrates an apparatus (500) for the catalytic decomposition of nitrous oxide. It is one of the best mode apparatus (207) to be used in the inventive system, i.e. for the further processing of nitrous oxide which is desorbed from adsorption units loaded with nitrous oxide emanating from air exhaled by patients. The apparatus (500) comprises as a rule a main flow line (502) for the process flow. Along the flow line there are an inlet arrangement (503), an outlet arrangement (504) and between these arrangements a decomposition chamber (507).

### The inlet arrangement comprises in the downstream direction

a) The inlet port (505,505a+505b). For maternity care during delivery, when a mother alternate between inhaling a nitrous oxide mixture and oxygen or air, there may be one inlet port (505a N₂O) for nitrous oxide (501a) and one (505b O₂) for oxygen/air (501b). There are also corresponding branch conduits (502a and 502b, respectively) and the proper function (535) for merging the two branch conduits to the common main flow line (502). This is similar to common inlet/outlet port IP_{ads}/OP_{des} (414a/415b) of figure 4.
b) A flow changing function FCF (523), which preferably is a blower, and preferably is placed in a gas proof box (536) in order to prevent leakage of nitrous oxide to ambient atmosphere. This FCF function is capable of giving at least two or at least three preset main levels of flow velocity in the main flow line: One for conditioning the apparatus prior to the inlet of nitrous oxide, one for running the actual decomposition and desorption in a connected adsorption unit and one for maintaining flow through a connected desorption unit after the heater (422b) of the adsorption unit has been turned off.
c) A flow sensor (525), preferably a flow meter. Measured flow values are used to control the flow changing function (523) to maintain flow at a preselected level.
d) A heating arrangement (515a+b) comprising
   i) heat exchanger (515a) in which hot gas which is leaving the decomposition chamber (507) is used to heat incoming gas to be processed in the decomposition chamber (507), and
   ii) a heater (515b) which advantageously i) is placed in a gap (534a, distributor function) close to the upstream end of the decomposition chamber (507), and i) has a temperature sensor (529), preferably designed as a safety thermostat, and.

The two separate inlet conduits (502a,b) with the merger function (535) may be placed outside the inventive apparatus or does not need to be present if the patient is only using one face mask or if the gas to be processed in the apparatus does not contain exhalation air. Nitrous oxide may for instance be desorbed from an adsorbent previously loaded with nitrous oxide by passing exhalation air containing nitrous oxide through the adsorbent. See also discussion with respect to figure 4.

### The decomposition chamber (507) comprises

e) A porous bed of catalyst material (508) and a heat neutralizing medium (509), e.g. a heat absorbing material, and possibly temperature sensors (527a,b,c). The catalyst material and the heat neutralizing medium are in intimate heat transfer contact. See our international patent application filed in parallel with this application (given above).

### The outlet arrangement comprises

f) A gap (534b) (collector function) immediately downstream of the decomposition chamber (507).
g) The heat exchanger discussed as item (d) above (515a) will at this position work as a downstream cooling arrangement (517a) by transferring heat in gas which exits the decomposition chamber to incoming gas which is about to enter the decomposition chamber.
h) A second downstream cooling arrangement (517b) which comprises an inlet conduit (519) for a cooling gas (520, preferably air) along which there is a blower (521) and a mixing function. This function may be a tube having a larger diameter than the flow line upstream of the mixing function and open at its downstream end.
i) Preferably a temperature sensor (528) which is placed between the inlet conduit (519) and the outlet port (506). This function is controlled by the temperature sensor (528) to increase or decrease the inlet flow of cooling gas via conduit (519) in response to an increase or a decrease, respectively, in the temperature measured by sensor (528) (relative to a desired preset temperature).
j) The outlet port (506)

As described elsewhere in this specification the various functions described above may be placed in a common housing (537). Parts/functions which will become warmed up during use of the apparatus should be properly heat insulated by the appropriate heat insulating material (518).

The apparatus may be equipped with wheels to support mobility. Proper face masks for inhalation of gas containing nitrous oxide and/or oxygen/air, respectively, as well as gas tubes containing these gases may be connected and/or carried to/by a mobile unit which contains the inventive apparatus.

### EXPERIMENTAL PART

### EXAMPLE 1

### Instrumentation and chemicals

*Gas mixture:* 50% nitrous oxide 50% oxygen from tubes.
*Spectrometer:* Sick IR for detecting nitrous oxide at the outlet end of the column
*Column*/*reactor*: Tube with a height of 20 cm, upward flow 3 L/min, three thermo elements placed i the adsorbent with a first element at 1 cm distance from the inlet end (top), a second element at the half-height, and a third element at the outlet 18 cm from the outlet end (bottom).
*Adsorbent material:* 1275 g Sylobead ®MS S 624 (Grace & Co, Cambridge, MA USA)
*Temperature:* Room temperature. Adsorption temperature 120-200°C.

### Results

*Adsorption:* Break-through at the outlet of the column occurred after 46 min. The total capacity of the column was determined by weighing to 74 g. During the experiment the temperature at the inlet (1 cm upstream of the inlet) had increased from 28°C to 41°C after 10 min and at the outlet (18 cm upstream of the inlet) from 30°C to 83°C after 30 min. The reason for the temperature increase is that adsorption heat is released. It was also noted that the temperature increase 1 cm upstream of the inlet was stopped at 41°C. This is interpreted as saturation with nitrous oxide at this part of the adsorbent. Adsorption heat was successively released when the adsorption front moved upwards (heat front). This temperature increase stopped at the outlet (and also in the whole adsorbent) when the adsorbent was fully saturated. This is an indication that release of adsorption heat potentially might be useful for indicating proceeding saturation degrees for this kind of adsorbents.
*Desorption:* The column was after adsorption heated to 120-200°C by an air flow of 10 mL/min passing through the adsorbent with simultaneous IR measurement of nitrous oxide at the outlet. After 30 minutes nitrous oxide could no longer be detected at the outlet. These experiments were repeated 5 times with essentially the same results.
It is intended therefore that the invention embraces those equivalents within the scope of the claims which follow from this.

Finally, it should be understood that the following aspects and embodiments. embodiments are related:
A1. A through-flow adsorption unit, characterized in that IPads coincides with OPdes and/or OPads coincides with IPdes.
A2. The unit according to A1, characterized in that there between each inlet port IPads (314a) and IPdes (315a) and the chamber (316a) there is an inlet conduit (316a and 317a, respectively) and/or between each outlet port OPdes (315b) and/or OPads (314b) and the chamber (316a) there is an outlet conduit (317b and 317b, respectively).
A3. The unit according to A1 or A2, characterized in that said heating function (322) is placed between the inlet port IPdes (315a) for the desorption flow and the adsorbent (313a).
A4. The unit according to A3, characterized in that there is a cooling function placed downstream of the adsorbent for cooling the warm desorption flow leaving the adsorbent, said cooling function preferably being part of a heat exchanger (422a) the heating function of which is used for heating of desorbing gas upstream of the adsorbent (413a).
A5. The unit according to any of A1-A4, characterized in that one of said flow changing function is a flow changing function FCFads (323,423) for changing the adsorption flow through the unit.
A6. The unit according to any of A1-A5, characterized in that one of said flow changing function is a flow changing function FCFdes (323) for changing the desorption flow through the unit.
A7. The unit according to A5-A6 in combination with A2, characterized in that
   a) said flow changing function FCFads (323,423) is associated with said inlet conduit or said outlet conduit for the adsorption flow, and/or
   b) said flow changing function FCFdes (323) is associated with said inlet conduit or said outlet conduit for the desorption flow.
A8. The unit according to any of A1-A7, characterized in that said one or more sensors (308a,b,408a,b,c) are based on measuring the amount of nitrous oxide on the adsorbent (213a,313a) during and/or after adsorption.
A9. The unit according to any of claims A1-A8, characterized in that
   A) the adsorbent (313a,413a) has been selected amongst adsorbents for which the adsorption of nitrous oxide is exothermic thereby developing measurable changes in temperature where adsorption is on-going indicating the advancing front of adsorption, and
   B) one or more temperature sensors (308a,b,c,408a,b,c) which
      a) are capable of measuring changes in temperature in the adsorbent (313a413a) caused by adsorption of nitrous oxide, and
      b) are placed at different predetermined longitudinal positions between the inlet end and the outlet end of the adsorbent (313a,413a).

## Claims

1. A system for
I) collecting nitrous oxide in air exhaled by an individual inhaling nitrous oxide via a face mask arrangement (= face mask) (204) having an outlet OLₘₐₛₖ (204) for exhalation, and
II) delivering the nitrous oxide collected in (I) to an inlet port IPₐₚₚ (217) of an apparatus for further processing of nitrous oxide,
wherein the system comprises a pool of one or more mutually replaceable through-flow adsorption units (201), each of which comprises a nitrous oxide reversible adsorbent (213a) placed in a through-flow chamber (213) and has:
i) an inlet port IP_{ads} (214a) for inlet of exhalation air (adsorption flow),
ii) an outlet port OP_{ads} (214b) for discharging gas processed in the chamber to a waste recipient (205),
iii) an inlet port IP_{des} (215a) for inlet of a desorbing gas (206) (desorption flow), and
iv) an outlet port OP_{des} (215b) for discharging desorbing gas from the adsorbent, together with
A) a docking arrangement DA_{ads} (202) which is associated with adsorption of nitrous oxide and comprises a connection C1_{ads} (219a) for connecting the outlet port OLₘₐₛₖ (212) to the inlet port IP_{ads} (214a), and
B) a docking arrangement DA_{des} (203) which is associated with desorption of nitrous oxide and comprises a connection C2_{des} (220b) for connecting the outlet port OP_{des} (215b) to the inlet port IPₐₚₚ (217)
**characterized in that** the system is associated with a measuring arrangement which comprises one or more sensors (208a,b,308a,b,c) which are based on measuring the amount of nitrous oxide on the adsorbent during and/or after adsorption,
and **in that**
A) the adsorbent in the adsorption units is selected amongst adsorbents for which the adsorption of nitrous oxide is exothermic developing measurable changes in temperature where adsorption is ongoing indicating the advancing front of adsorption, and
B) one or more temperature sensitive sensors (308a,b,c) which
a) are capable of measuring changes in temperature in the adsorbent caused by adsorption of nitrous oxide, and
b) are placed at different predetermined longitudinal positions between an inlet end (313c) and an outlet end (313b) of the adsorbent.

2. The system of claim 1, **characterized in that** the individual adsorption units (301) comprises a carrier function (318) to support their mobility.

3. The system of any of claims 1-2, **characterized in that** the flow direction for desorbing flow through the adsorption unit is opposite to, or has the same direction as the flow direction of absorption flow (exhalation air).

4. The system of any of claims 1-3, **characterized in** IP_{ads} (314a) coinciding with OP_{des} (315b) and/or OP_{ads} (314b) coinciding with IP_{des} (315a)

5. The system of any of claims 1-4, **characterized in** comprising a heating arrangement comprising one or more functions (222,322) for heating the adsorbent of the individual adsorption units.

6. The system of any of claims 1-5, **characterized in**
a) one of said heating functions (222) being present on said docking arrangement DA_{des} (203) and placed upstream of the connection C1_{des} (220a) and/or
b) one of said heating functions (322) being present on every adsorption unit (301) and placed between the inlet port IP_{des} (315a) and the adsorbent (313a).

7. The system of any of claims 1-6, **characterized in** being associated with a flow changing function FCF_{ads} (223a,324) for changing adsorption flow through the adsorption unit (201,301), and/or a flow changing function FCF_{des} (223b,324) for changing the desorption flow through the adsorption unit (201,301).

8. The system of claims 7, **characterized in**
a) said flow changing function FCF_{des} being associated with the apparatus for further processing (207) and/or with the source (206) of desorbing gas, and/or
b) said flow changing function FCF_{ads} being associated with the face mask (204) and/or an inlet conduit (316a) of an adsorption unit.

9. The system according to any of claims 1-8, **characterized in** comprising a logging arrangement (310a+b) comprising a memory (310a+b) for storing a) capacity data and/or flow capacity data and/or sum of running times for adsorption for at least the latest time of use for the individual adsorbents of the pool etc, and possibly also corresponding initial values for freshly prepared adsorbents of the pool, and/or b) preset limit values for disqualification/qualification of the individual adsorption units for repetitive use in the system.

## Patentansprüche

1. System zum
I) Sammeln von Distickstoffoxid (Lachgas) in Luft, die von einem Individuum ausgeatmet wird, welches Distickstoffoxid über eine Gesichtsmaskenanordnung (= Gesichtsmaske) (204) inhaliert, mit einem Auslass OLₘₐₛₖ (204) zur Ausatmung, und
II) Übermitteln des in (I) gesammelten Distickstoffoxids an eine Einlassöffnung IPₐₚₚ (217) einer Vorrichtung zur Weiterverarbeitung von Distickstoffoxid,
wobei das System einen Vorrat von einer oder mehreren gegeneinander austauschbaren Durchflussadsorptionseinheiten (201) umfasst, die jeweils ein reversibles Distickstoffoxid-Adsorbens (213a) umfassen, das in einer Durchflusskammer (213) platziert ist, und aufweist:
i) eine Einlassöffnung IP_{ads} (214a) zum Einlassen der Ausatmungsluft (Adsorptionsfluss),
ii) eine Auslassöffnung OP_{ads} (214b) zum Abgeben von Gas, das in der Kammer verarbeitet wurde, an einen Abfallempfänger (205),
iii) eine Einlassöffnung IP_{des} (215a) zum Einlassen eines desorbierenden Gases (206) (Desorptionsfluss), und
iv) eine Auslassöffnung OP_{des} (215b) zum Abgeben des desorbierenden Gases von dem Adsorbens, zusammen mit
A) einer Docking-Anordnung DA_{ads} (202), die zu der Adsorption von Distickstoffoxid gehört und eine Verbindung C1_{ads} (219a) zum Verbinden der Auslassöffnung OLₘₐₛₖ (212) mit der Einlassöffnung IP_{ads} (214a) umfasst, und
B) eine Docking-Anordnung DA_{des} (203), die zu der Desorption von Distickstoffoxid gehört und eine Verbindung C2_{des} (220b) zum Verbinden der Auslassöffnung OP_{des} (215b) mit der Einlassöffnung IPₐₚₚ (217) umfasst,
**dadurch gekennzeichnet, dass** zu dem System eine Messanordnung gehört, die einen oder mehrere Sensoren (208a, b, 308a, b) umfasst, die auf der Messung der Menge an Distickstoffoxid auf dem Adsorbens während und/oder nach der Adsorption basieren, und dass
A) das Adsorbens in den Adsorptionseinheiten ausgewählt ist aus Adsorbentien, für die die Adsorption von Distickstoffoxid exotherm ist, wodurch messbare Temperaturänderungen entwickelt werden, während die Adsorption läuft, die das Voranschreiten der Adsorptionsfront anzeigen, und
B) einen oder mehrere temperaturempfindliche Sensoren (308a, b, c), die
a) in der Lage sind, Temperaturänderungen in dem Adsorbens zu messen, die durch Adsorption von Distickstoffoxid verursacht werden, und
b) in unterschiedlichen vorbestimmten Längspositionen zwischen einem Einlassende (313c) und einem Auslassende (313b) des Adsorbens platziert werden.

2. System nach Anspruch 1, **dadurch gekennzeichnet, dass** die einzelnen Adsorptionseinheiten (301) eine Trägerfunktion (318) umfassen, um ihre Mobilität zu unterstützen.

3. System nach einem der Ansprüche 1-2, **dadurch gekennzeichnet, dass** die Flussrichtung für den desorbierenden Fluss durch die Adsorptionseinheit hindurch entgegengesetzt zu der Flussrichtung des Absorptionsflusses (Ausatmungsluft) ist oder die gleiche Richtung wie diese aufweist.

4. System nach einem der Ansprüche 1-3, **dadurch gekennzeichnet, dass** IP_{ads} (314a) mit OP_{des} (315b) zusammenfällt und/oder OP_{ads} (314b) mit IP_{des} (315a) zusammenfällt.

5. System nach einem der Ansprüche 1-4, **dadurch gekennzeichnet, dass** es eine Heizanordnung umfasst, die eine oder mehrere Funktionen (222, 322) zum Erwärmen des Adsorbens in den einzelnen Adsorptionseinheiten umfasst.

6. System nach einem der Ansprüche 1-5, **dadurch gekennzeichnet, dass**
a) eine der Heizfunktionen (222) auf der Docking-Anordnung DA_{des} (203) vorhanden ist und stromaufwärts der Verbindung C1_{des} (220a) platziert ist, und/oder
b) eine der Heizfunktionen (322) auf jeder Adsorptionseinheit (301) vorhanden ist und zwischen der Einlassöffnung IP_{des} (315a) und dem Adsorbent (313a) platziert ist.

7. System nach einem der Ansprüche 1-6, **dadurch gekennzeichnet, dass** zu ihm eine Flussänderungsfunktion FCF_{ads} (223a, 324), um den Adsorptionsfluss durch die Adsorptionseinheit (201, 301) zu ändern, und/oder eine Flussänderungsfunktion FCF_{des} (223b, 324) gehört, um den Desorptionsfluss durch die Adsorptionseinheit (201, 301) hindurch zu ändern.

8. Verfahren nach Anspruch 7, **dadurch gekennzeichnet, dass**
a) die Flussänderungsfunktion FCF_{des} zu der Vorrichtung zur Weiterverarbeitung (207) und/oder zu der Quelle (206) des desorbierenden Gases gehört, und/oder
b) die Flussänderungsfunktion FCF_{ads} zu der Gesichtsmaske (204) und/oder einer Einlassleitung (316a) einer Adsorptionseinheit gehört.

9. System nach einem der Ansprüche 1-8, **dadurch gekennzeichnet, dass** es eine Protokollieranordnung (310a+b) umfasst, die einen Speicher (310a+b) umfasst, um a) Kapazitätsdaten und/oder Flusskapazitätsdaten und/oder eine Summe der Laufzeiten für die Adsorption für mindestens den spätesten Nutzungszeitpunkt für die einzelnen Adsorbentien des Vorrats, usw. und möglicherweise auch entsprechende Anfangswerte für frisch hergestellte Adsorbentien des Vorrats zu speichern, und/oder b) vorgegebene Grenzwerte zur Aussonderung/Qualifikation der einzelnen Adsorptionseinheiten zur wiederholten Verwendung in dem System zu speichern.

## Revendications

1. Système pour
I) collecter de l'oxyde nitreux dans l'air exhalé par un individu inhalant de l'oxyde nitreux via un agencement de masque facial (= masque facial) (204) comportant une sortie OLₘₐₛₖ (204) pour l'expiration, et
II) acheminer l'oxyde nitreux recueilli en (I) à un orifice d'entrée IPₐₚₚ (217) d'un appareil pour le traitement ultérieur de l'oxyde nitreux,
dans lequel le système comprend un groupe d'une ou plusieurs unités d'adsorption (201) à flux traversier interchangeables, dont chacune comprend un adsorbant réversible (213a) d'oxyde nitreux placé dans une chambre (213) à flux traversier et comporte :
i) un orifice d'entrée IP_{ads} (214a) pour l'entrée de l'air expiré (flux d'adsorption),
ii) un orifice de sortie OP_{ads} (214b) pour dégager le gaz traité dans la chambre vers un récipient à déchets (205),
iii) un orifice d'entrée IP_{des} (215a) pour l'entrée d'un gaz désorbeur (206) (flux de désorption), et
iv) un orifice de sortie OP_{des} (215b) pour évacuer le gaz désorbeur de l'adsorbant, conjointement avec :
A) un agencement de connexion DA_{ads} (202) qui est associé à l'adsorption d'oxyde nitreux et comprend une connexion C1_{ads} (219a) pour raccorder l'orifice de sortie OLₘₐₛₖ (212) à l'orifice d'entrée IP_{ads} (214a), et
B) un agencement de connexion DA_{des} (203) qui est associé à l'adsorption d'oxyde nitreux et comprend une connexion C2_{des} (220b) pour raccorder l'orifice de sortie OP_{des} (215b) à l'orifice d'entrée IPₐₚₚ (217), **caractérisé en ce que** le système est associé à un agencement de mesure qui comprend un ou plusieurs capteurs (208a, b ; 308a, b, c) qui reposent sur la mesure de la quantité d'oxyde nitreux sur l'adsorbant pendant et/ou après l'adsorption, et
**en ce que** :
A) l'adsorbant dans les unités d'adsorption est choisi parmi les adsorbants pour lesquels l'adsorption d'oxyde nitreux est exothermique et développe des modifications mesurables de température là où l'adsorption est en cours, indiquant l'avancement du front d'adsorption, et
B) un ou plusieurs capteurs (308a, b, c) sensibles à la température qui
a) sont capables de mesurer les modifications de température dans l'adsorbant provoquées par l'adsorption d'oxyde nitreux, et
b) sont placés à différentes positions longitudinales prédéterminées entre une extrémité d'entrée (313c) et une extrémité de sortie (313b) de l'adsorbant.

2. Système selon la revendication 1, **caractérisé en ce que** les unités individuelles d'adsorption (301) comprennent une fonction de support (318) pour soutenir leur mobilité.

3. Système selon la revendication 1 ou 2, **caractérisé en ce que** le sens d'écoulement du flux désorbeur à travers l'unité d'adsorption est contraire à, ou a le même sens que le sens d'écoulement du flux d'absorption (l'air expiré).

4. Système selon l'une quelconque des revendications 1 à 3, **caractérisé en ce que** IP_{ads} (314a) coïncide avec OP_{des} (315b) et/ou OP_{ads} (314b) coïncide avec IP_{des} (315a).

5. Système selon l'une quelconque des revendications 1 à 4, **caractérisé en ce qu'**il comprend un dispositif de chauffage comprenant une ou plusieurs fonctions (222, 322) pour chauffer l'adsorbant des unités individuelles d'adsorption.

6. Système selon l'une quelconque des revendications 1 à 5, **caractérisé en ce que**
a) une desdites fonctions de chauffage (222) est présente sur ledit agencement de connexion DA_{des} (203) et placé en amont de la connexion C1_{des} (220a) et/ou
b) une desdites fonctions de chauffage (322) est présente sur chaque unité d'adsorption (301) et placée entre l'orifice d'entrée IP_{des} (315a) et l'adsorbant (313a).

7. Système selon l'une quelconque des revendications 1 à 6, **caractérisé en ce qu'**il est associé à une fonction de changement de flux FCF_{ads} (223a, 324) pour modifier le flux d'adsorption à travers l'unité d'adsorption (201, 301), et/ou une fonction de changement de flux FCF_{des} (223b, 324) pour modifier le flux de désorption à travers l'unité d'adsorption (201, 301).

8. Système selon la revendication 7, **caractérisé en ce que**
a) ladite fonction de changement de flux FCF_{des} est associée à l'appareil pour un traitement ultérieur (207) et/ou à la source (206) de gaz désorbeur, et/ou
b) ladite fonction de changement de flux FCF_{ads} est associée au masque facial (204) et/ou à un conduit d'entrée (316a) d'une unité d'adsorption.

9. Système selon l'une quelconque des revendications 1 à 8, **caractérisé en ce que** il comprend un agencement de journalisation (310a+b) comprenant une mémoire (310a+b) pour stocker a) des données de capacité et/ou des données de capacité d'écoulement et/ou la somme des durées de fonctionnement de l'adsorption pour au moins la plus récente période d'utilisation pour les adsorbants individuels du groupe, etc., et éventuellement les valeurs initiales correspondantes pour les adsorbants fraîchement préparés du groupe, et/ou b) des valeurs limites prédéfinies pour la disqualification/qualification des unités individuelles d'adsorption pour une utilisation répétitive dans le système.
